# EUROPÄISCHE PATENTANMELDUNG

(11) **EP 4 043 538 A1**
(43) Veröffentlichungstag der Anmeldung: **17.08.2022**
(21) Anmeldenummer: 21157138.5
(22) Anmeldetag: 15.02.2021
(51) Int. Cl.: C10G 9/36, C10G 9/00

(54) **VERFAHREN UND ANLAGE ZUR HERSTELLUNG VON ETHYLEN UND/ODER ANDEREN OLEFINEN DURCH STEAMCRACKEN**

(71) Anmelder: Linde GmbH, 82049 Pullach (DE); BASF SE, 67056 Ludwigshafen am Rhein (DE)
(72) Erfinder: Keller, Benedikt, 80339 München (DE); Brehm, Peter, 85716 Unterschleißheim (DE); Prunner, Clemens, 85646 Neufarn (DE); Reyneke, Hendrik, 81479 München (DE); Kamann, Martin, 82041 Oberhaching (DE); Garbe, Gunter, 67688 Rodenbach (DE); Keck, Daniel, 68775 Ketsch (DE); Schietekat, Carl, 9100Z SINT-NIKLAAS (BE); Sperber, Axel, 67069 Ludwigshafen (DE); Weck, Alexander, 67126 Hochdorf-Assenheim (DE)
(74) Vertreter: DehnsGermany Partnerschaft von Patentanwälten

(57) **Zusammenfassung**

Ein Verfahren (100) zur Herstellung von Ethylen und/oder anderen Olefinen durch Steamcracken wird vorgeschlagen, bei dem ein oder mehrere Cracker (10) mit einem paraffinhaltigen Feed beschickt werden und dem einen oder mehreren Crackern (10) ein Rohgas entnommen wird, wobei das Rohgas zumindest zum Teil einer Aufbereitung (20) unterworfen wird, die eine Verdichtung (22) und eine thermische Trennung (23) unter Verwendung eines C2-Kältemittels und eines C3-Kältemittels umfasst, wobei für die Rohgasverdichtung (22) ein Rohgasverdichter (CGC) verwendet wird, wobei das Ethylenkältemittel unter Verwendung eines C2-Kältemittelverdichters (ERC) verdichtet wird, und wobei das Propylenkältemittel unter Verwendung eines C3-Kältemittelverdichters (PRC) verdichtet wird. Hierbei ist vorgesehen, dass der Rohgasverdichter (CGC), der C2-Kältemittelverdichter (ERC) und der C3-Kältemittelverdichter (PRC) jeweils zumindest zu einem Teil unter Verwendung eines elektrischen Hauptantriebs (M) betrieben werden. Eine entsprechende Anlage ist ebenfalls Gegenstand der Erfindung.

## Beschreibung

Die Erfindung betrifft ein Verfahren und eine Anlage zur Herstellung von Ethylen und/oder von anderen Olefinen durch Steamcracken gemäß den Oberbegriffen der jeweiligen unabhängigen Patentansprüche.

### Hintergrund der Erfindung

Verfahren und Anlagen zum Steamcracken von Kohlenwasserstoffen sind beispielsweise im Artikel "Ethylene" in Ullmann's Encyclopedia of Industrial Chemistry, Onlineausgabe, 15. April 2009, DOI: 10.1002/14356007.a10_045.pub2, beschrieben. Das Steamcracken (engl. Steam Cracking, im Deutschen auch als Dampfspalten bezeichnet) wird vorwiegend zur Gewinnung von kurzkettigen Olefinen wie Ethylen und Propylen, Diolefinen wie Butadien oder von Aromaten eingesetzt, ist jedoch nicht auf die Gewinnung solcher Verbindungen beschränkt.

Beim Steamcracken werden Komponentengemische (auch als Spaltgase oder Rohgase bezeichnet) erhalten, die zur Gewinnung der gewünschten Einzelkomponenten geeigneten Aufbereitungssequenzen unterworfen werden. Typischerweise erfolgt dabei in einem ersten Abschnitt (engl. Front-End Section) einer entsprechenden Aufbereitungssequenz eine Entfernung schwerer Verbindungen, falls vorhanden, und danach eine sogenannte Rohgasverdichtung, Sauergasentfernung und Trocknung. Nach der Aufbereitung in dem ersten Abschnitt erfolgt eine Fraktionierung, in der durch thermische Trennverfahren unter Einsatz von Ethylen- bzw. C2-Kältemittel und Propylen- bzw. C4-Kältemittel Fraktionen gebildet und bei Bedarf weiter aufgetrennt werden. Zu Details sei auf den erwähnten Artikel "Ethylene" in Ullmann's Encyclopedia of Industrial Chemistry, insbesondere die Abschnitte 5.3.2.1, "Front-End Section", und 5.3.2.2, "Hydrocarbon Fractionation Section", verwiesen.

In einer Ausgestaltung einer entsprechenden Fraktionierung, die auch im Zusammenhang mit der vorliegenden Erfindung zum Einsatz kommen kann, erfolgt in der Fraktionierung zunächst eine Trennung von Kohlenwasserstoffen mit zwei Kohlenstoffatomen und tiefer siedenden Komponenten wie Methan und Wasserstoff von Kohlenwasserstoffen mit drei Kohlenstoffatomen und höher siedenden Verbindungen. Ein derartiger Schritt wird üblicherweise auch als Deethanisierung bezeichnet, die Ausgestaltung einer entsprechenden Fraktionierung als "Deethanizer-First"- oder "Frontend-Deethanizer"-Verfahren.

Die in der Deethanisierung gasförmig erhaltene Fraktion von Kohlenwasserstoffen mit zwei Kohlenstoffatomen und tiefer siedenden Komponenten kann einer weiteren Trennung zugeführt werden, in der die Kohlenwasserstoffe mit zwei Kohlenstoffatomen von den weiter enthaltenen tiefer siedenden Komponenten abgetrennt werden. Ein derartiger Schritt wird auch als Demethanisierung bezeichnet. In einem "Deethanizer-First"- oder "Frontend-Deethanizer"-Verfahren ist also die Demethanisierung der Deethanisierung nachgeschaltet.

In alternativen Verfahren können die Schritte der Deethanisierung und der Demethanisierung auch in umgekehrter Reihenfolge durchgeführt werden. Man spricht dann von "Demethanizer-First"- oder "Frontend-Demethanizer"-Verfahren. Weitere Verfahrensvarianten sind in der erwähnten Fachliteratur beschrieben.

Im Zuge entsprechender Aufarbeitungssequenzen kommen an unterschiedlichen Stellen Verdichter zum Einsatz. Insbesondere wird dabei die Rohgasverdichtung unter Verwendung eines Rohgasverdichter (engl. Cracked Gas Compressor, CGC) vorgenommen und bei der Bereitstellung der Ethylen- bzw. C2-Kältemittel und Propylen- bzw. C3-Kältemittel kommen ein sogenannter Ethylenkältemittelverdichter (engl. Ethylene Refrigerant Compressor, ERC) und ein sogenannter Propylenkältemittelverdichter (engl. Propylene Refrigerant Compressor, ERC) zum Einsatz. Nachfolgend werden diese Begriffe verwendet, wenngleich in einem Ethylenkältemittelverdichter auch ggf. Ethan und in einem Propylenkältemittelverdichter auch ggf. Propan verdichtet werden kann. Produktfraktionen der thermischen Trennung können einer Verdichtung unter Verwendung weiterer Verdichter, sogenannter Produktverdichter, unterworfen werden.

Die Erfindung stellt sich die Aufgabe, entsprechende Verfahren und Anlagen zu verbessern und dabei insbesondere auch an das jeweilige Energieangebot am Standort der Anlage anpassbar auszugestalten.

### Offenbarung der Erfindung

Diese Aufgabe wird durch ein Verfahren und eine Anlage zur Herstellung von Ethylen und/oder anderen Olefinen durch Steamcracken mit den Merkmalen der jeweiligen unabhängigen Patentansprüche gelöst. Ausgestaltungen sind jeweils Gegenstand der abhängigen Patentansprüche und der nachfolgenden Beschreibung.

Insgesamt schlägt die vorliegende Erfindung ein Verfahren zur Herstellung von Ethylen und/oder anderen Olefinen durch Steamcracken vor, bei dem ein oder mehrere Cracker, also Spaltöfen, die in der üblichen Weise mit Konvektions- und Strahlungszone ausgebildet sein können, mit einem paraffinhaltigen Feed wie Naphtha oder Ethan bzw. entsprechenden Gemischen oder beliebigen anderen, in der Fachwelt bekannten oder vorteilhaften Einsätzen beschickt werden und bei dem dem einen oder den mehreren Crackern ein Rohgas entnommen wird, wobei das Rohgas zumindest zum Teil einer Aufbereitung unterworfen wird, die eine Rohgasverdichtung und eine thermische Trennung unter Verwendung eines Ethan- und/oder Ethylenkältemittels (C2-Kältemittel) und eines Propan- und/oder Propylenkältemittels (C3-Kältemittel) umfasst. Die genannten Kältemittel können insbesondere zum Kondensieren von Gasgemischen, zum Aufkochen von Sumpfverdampfern von Trennkolonnen oder in entsprechenden Kopfkondensatoren eingesetzt werden.

Ist hier von einem "Ethan- und/oder Ethylenkältemittel" oder "C2-Kältemittel" bzw. einem "Propan- und/oder Propylenkältemittel" bzw. "C3-Kältemittel" die Rede, kann es sich um entsprechende Reinstoffe oder Gemische der genannten Komponenten handeln. Es können jeweils, typischerweise in geringeren Mengen von weniger als 10%, auch andere Komponenten enthalten sein.

Für die Rohgasverdichtung wird im Rahmen der vorliegenden Erfindung ein Rohgasverdichter verwendet, das C2-Kältemittel wird unter Verwendung einesC2-Kältemittelverdichters verdichtet, und das C3-Kältemittel wird unter Verwendung einesC3-Kältemittelverdichters verdichtet. Zu weiteren Details eines entsprechenden Verfahrens, das auch weiter unten nochmals exemplarisch erläutert wird, sei auf den eingangs zitierten Stand der Technik ausdrücklich verwiesen. Wie erwähnt, werden der C2-Kältemittelverdichter und der C3-Kältemittelverdichter vereinfacht auch als C2-Kältemittelverdichter (ERC) bzw. C3-Kältemittelverdichter (PRC) bezeichnet.

Erfindungsgemäß werden der Rohgasverdichter, der C2-Kältemittelverdichter und der C3-Kältemittelverdichter jeweils zumindest zu einem Teil unter Verwendung eines oder mehrerer elektrischer Antriebe betrieben.

Die erfindungsgemäß vorgeschlagene Betriebsweise bietet, wie erfindungsgemäß erkannt wurde, besondere Vorteile gegenüber aus dem Stand der Technik bekannten Antrieben, beispielsweise mittels Kondensations-Dampfturbinen nach API 612 oder anderen. Bei einer derartigen herkömmlichen Betriebsweise, deren nachfolgende Beschreibung die Erfindung in keiner Weise einschränken soll, wird beispielsweise unter Verwendung von Abwärme der Cracker Höchstdruckdampf (HHP-Dampf), d.h. Dampf auf einem Druckniveau von 90 bis 130 bar und einem Temperaturniveau von 450 bis 540 °C, bereitgestellt und zum Antrieb des Rohgasverdichters verwendet. Der Höchstdruckdampf wird dabei typischerweise über einen Hochdruckteil der Antriebsturbine des Rohgasverdichters entspannt. Ein signifikanter Anteil des Dampfes wird anschließend als Hochdruckdampf (HP-Dampf), d.h. Dampf auf einem Druckniveau von 35 bis 50 bar und einem Temperaturniveau von 250 bis 400 °C, extrahiert und den Antriebsturbinen desC2-Kältemittelverdichters und desC3-Kältemittelverdichters zugeführt. Je nach Bedarf weiterer Verbraucher werden zudem auch Mitteldruckdampf (MP-Dampf) bzw. Niederdruckdampf (LP-Dampf), also Dampf auf einem Druckniveau von 15 bis 25 bar und einem Temperaturniveau von 200 bis 250 °C bzw. Dampf auf einem Druckniveau von 3 bis 8 bar und einem Temperaturniveau von 150 bis 190 °C, erzeugt. Zur H ebung der Gesamtenergienbilanz kann zusätzlicher Dampf aus Hochdruckdampferzeugern oder von extern importiert werden. Ebenso kann hierbei ein Export von Dampf über die Anlagengrenze zur Bilanzierung vorgesehen sein.

Die energetische Bilanzierung des soeben beschriebenen herkömmlichen Verfahrens muss unter Beachtung der Restriktionen erfolgen, welche sich aus dem Leistungsbedarf der drei großen Verbraucher, d.h. des Rohgasverdichters, desC2-Kältemittelverdichters und des C3-Kältemittelverdichters, ergeben. Dies erfordert i.d.R. die Installation einer Hochdruckdampferzeugung umfassend einen entsprechenden Dampfkessel, um die Kopplung von Höchst- und Hochdruckdampfbedarf bilanztechnisch zu lösen. Dies erhöht den baulichen Aufwand. Zudem bleibt die Kondensationswärme des Dampfes weitgehend ungenutzt, vielmehr wird der Abdampf der Turbinen typischerweise mittels Kühlwasser mit hohem apparativem Aufwand kondensiert. Höchst- und Hochdruckdampferzeugung sind mit erheblichen Kohlendioxidemissionen verbunden, eine Einbindung alternativer (ggfs. kohlendioxidneutraler) Energie für den Antrieb der Verdichter ist herkömmlicherweise nicht vorgesehen oder möglich.

Durch den Einsatz der vorliegenden Erfindung ergibt sich durch den durch die Verwendung der elektrischen Antriebe sehr viel größeren Unabhängigkeit beim Betrieb der Verdichter eine Flexibilisierung der Ethylenerzeugung bzw. der Erzeugung anderer Olefine hinsichtlich der Dampf- und Kohlendioxidbilanzierung. Insbesondere steht die starre Kopplung der Höchstdruckdampfnutzung in der Rohgasverdichtung und der anschließenden Hochdruckdampfnutzung in der Verdichtung der Kältemittel im Rahmen der vorliegenden Erfindung der Flexibilisierung nicht mehr im Wege.

Gemäß einer besonders bevorzugten Ausgestaltung der vorliegenden Erfindung wird unter Verwendung von Abwärme des oder der Cracker weiterhin Höchstdruckdampf bereitgestellt. Dieser wird jedoch vorteilhafterweise zumindest zu einem Teil aus der Anlage exportiert und/oder zumindest teilweise ohne Verwendung zum Antrieb des Rohgasverdichters, des C2-Kältemittelverdichters und des C3-Kältemittelverdichters als Wärmequelle für andere Verfahrensschritte verwendet.

Der Höchstdruckdampf kann in einer besonders bevorzugten Ausgestaltung der vorliegenden Erfindung oder alternativ zu den erfindungsgemäß vorgeschlagenen Maßnahmen in einer Adaptionseinheit (sogenannte Let-Down-Station) hinsichtlich Druck und/oder Temperatur für den erwähnten Export und/oder den Verwendungszweck angepasst werden. In der Adaptionseinheit kann auch insbesondere anfallende Kondensationswärme für weitere Zwecke, beispielsweise zur Einspeisung in ein Fernwärmenetz, genutzt werden.

Das erfindungsgemäß eingesetzte Verfahren kann gemäß einer besonders bevorzugten Ausgestaltung ohne Verwendung eines Hochdruckdampfkessels durchgeführt werden. Mit anderen Worten kann durch den Einsatz der vorliegenden Erfindung ein entsprechender Hochdruckdampfkessels in Entfall kommen, so dass sich der Bedarf an fossilen Energieträgern verringert und damit die Kohlendioxidbilanz verbessert. Grundsätzlich kann im Rahmen der vorliegenden Erfindung an beliebigen Stellen eine Einbindung von kohlendioxidneutraler Energie erfolgen, wodurch sich eine erhebliche Flexibilisierung hinsichtlich des Kohlendioxid-Footprints ergibt.

Der Rohgasverdichter kann im Rahmen der vorliegenden Erfindung insbesondere zwei serielle Verdichterstränge umfassen, also individuell antreibbare bauliche Einheiten, wobei jeder der Verdichterstränge des Rohgasverdichters sowie der C2-Kältemittelverdichter und der C3-Kältemittelverdichter jeweils mittels zumindest teilweise identische Leistungsmerkmale aufweisender elektrischer Antriebe betrieben werden. Vorteilhafterweise können die Antriebe auch im Wesentlichen baulich identisch ausgebildet sein. Auf diese Weise erhöht sich die Anzahl an Gleichteilen und die Erstellung einer entsprechenden Anlage wird im Sinne eines Standardisierungskonzepts deutlich verbessert.

Die zumindest teilweise identische Leistungsmerkmale aufweisenden elektrischen Antriebe können dabei insbesondere als baugleiche drehzahlvariable Antriebe bereitgestellt werden und jeweils über Frequenzumrichter gespeist werden. Auf diese Weise lässt sich eine entsprechende Standardisierung bei gleichzeitiger Flexibilität in der Drehzahl erreichen. Hierbei können insbesondere vier Frequenzumrichter bereitgestellt sein, von denen zu jedem Zeitpunkt des Anlagenbetriebs zwei oder auch alle vier zur Speisung der elektrischen Antriebe eingesetzt werden können. Ein fünfter Frequenzumrichter kann aus Redundanzgründen bereitgehalten werden, insbesondere für notwendige Wartungs- und/oder Reparaturarbeiten.

Die vier Frequenzumrichter können dabei paarweise mit vollständiger Redundanz (2 x 100%) installiert werden wobei die beiden Frequenzumrichter jedes Paars im Teillastbetrieb (2 x 50%) parallel oder im im Umschaltbetrieb (abwechselnd) mit Vollast (jeweils 1 x 100%) betrieben werden können. Im Fall von zwei unabhängigen Versorgungsnetzen können die Frequenzumrichter vorteilhaft auf die beiden Netze verschaltet werden. Dieses Redundanzkonzept bietet sich insbesondere an, wenn Spannungseinbrüche/Ausfälle in den Versorgungsnetzen erwartet werden und Umschaltzeiten zur Erhöhung der Anlagenverfügbarkeit minimiert werden sollen.

Für leistungsstarke Antriebe jenseits der Leistungsgrenze eines Frequenzumrichters ist zudem ein 3 x 50% Redundanzkonzept denkbar, bei dem zwei parallel betriebene Frequenzumrichter die Summenleistung von 100% bereitstellen.

Ein besonderer Vorteil der vorliegenden Erfindung besteht auch darin, dass der Rohgasverdichter, der C2-Kältemittelverdichter und der C3-Kältemittelverdichter ebenerdig aufgestellt werden können, da keine Tischfundamente erfordernden Kondensatoren notwendig sind.

Vorteilhafterweise kann in dem Rohgasverdichter eine Zwischenkühlung zumindest zeitweise unter Verwendung von Fremdkälte vorgenommen werden, z.B. durch den Einsatz von Propan- oder Propylenkälte (C3-Kälte). Prinzipiell kann diese Zwischenkühlung vor jeder Stufe erfolgen. Eine Vorkühlung insbesondere der vierten Stufe ist besonders günstig, da hierdurch gleiche Leistung der ersten bis dritten Stufe einerseits und der vierten und fünften Stufe andererseits erreicht werden kann, unter Vermeidung unzulässig hoher Austrittstemperaturen. Dies ist insbesondere im Sinne der beschriebenen Standardisierung besonders günstig. Die Fremdkälte für die Zwischenkühlung kann im Rahmen der vorliegenden Erfindung unter Verwendung zumindest eines Teils des C2-Kältemittels und/oder des C3-Kältemittels bereitgestellt werden, indem entsprechendes Kältemittel aus den jeweiligen Kältemittelkreisläufen ausgekoppelt wird.

In Ausgestaltungen der vorliegenden Erfindung kann die erwähnte Zwischenkühlung entweder permanent oder aber immer (nur) dann unter Verwendung von Fremdkälte vorgenommen werden, wenn eine Kühlwassertemperatur und/oder Wassereinspritzung als nicht ausreichend und/oder nicht funktionsfähig erkannt wird. Hieraus ergibt sich die Möglichkeit, Betriebskosten zu optimieren.

Im Rahmen der vorliegenden Erfindung ist in einer besonders bevorzugten Ausgestaltung vorgesehen, die Antriebsleistungen der elektrischen Antriebe des Rohgasverdichters, des C2-Kältemittelverdichter und des C3-Kältemittelverdichters durch eine Verschiebung von Kälteleistung zwischen einem C2-Kältemmittelkreislauf, in dem das C2-Kältemittel eingesetzt wird, und eines C3-Kältemittelkreislaufs, in dem das C3-Kältemittel eingesetzt wird, und/oder durch die erläuterte Einbindung von C3-Kälte als zusätzliche Zwischenkühlung des Rohgasverdichters einzustellen, um den Leistungsbedarf möglichst zu vergleichmäßigen.

Die Erfindung erstreckt sich auch auf eine Anlage zur Herstellung von Ethylen und/oder anderen Olefinen durch Steamcracken, mit einem oder mehreren Crackern, die zur Beschickung mit einem paraffinhaltigen Feed und zur Bereitstellung eines Rohgases eingerichtet sind, wobei die Anlage dafür eingerichtet ist, das Rohgas zumindest zum Teil einer Aufbereitung zu unterwerfen, die eine Rohgasverdichtung und eine thermische Trennung unter Verwendung eines C2-Kältemittels und eines C3-Kältemittels umfasst, wobei für die Rohgasverdichtung ein Rohgasverdichter bereitgestellt ist, zur Verdichtung des C2-Kältemittels ein C2-Kältemittelverdichter bereitgestellt ist, und zur Verdichtung des C3-Kältemittels ein C3-Kältemittelverdichter bereitgestellt ist. Erfindungsgemäß sind zur Bereitstellung jeweils zumindest eines Teils der Antriebsleistung des Rohgasverdichters, des C2-Kältemittelverdichters und des C3-Kältemittelverdichter jeweils ein oder mehrere elektrische Antriebe bereitgestellt.

Zu Merkmalen und Vorteilen einer entsprechenden Anlage und vorteilhafter Ausgestaltungen hiervon sei auf die obigen Erläuterungen bezüglich des erfindungsgemäß vorgeschlagenen Verfahrens und seiner Ausgestaltungen ausdrücklich verwiesen, da diese in entsprechender Weise auch für die Anlage und deren Ausgestaltungen gelten. Dies ist insbesondere auch bei einer Anlage der Fall, die zur Durchführung eines Verfahrens eingerichtet ist, wie es oben in unterschiedlichen Ausgestaltungen erläutert wurde.

Die Erfindung wird nachfolgend unter Bezugnahme auf die beigefügte Zeichnung erläutert, welche eine Ausgestaltung der Erfindung veranschaulicht.

### Figurenbeschreibung

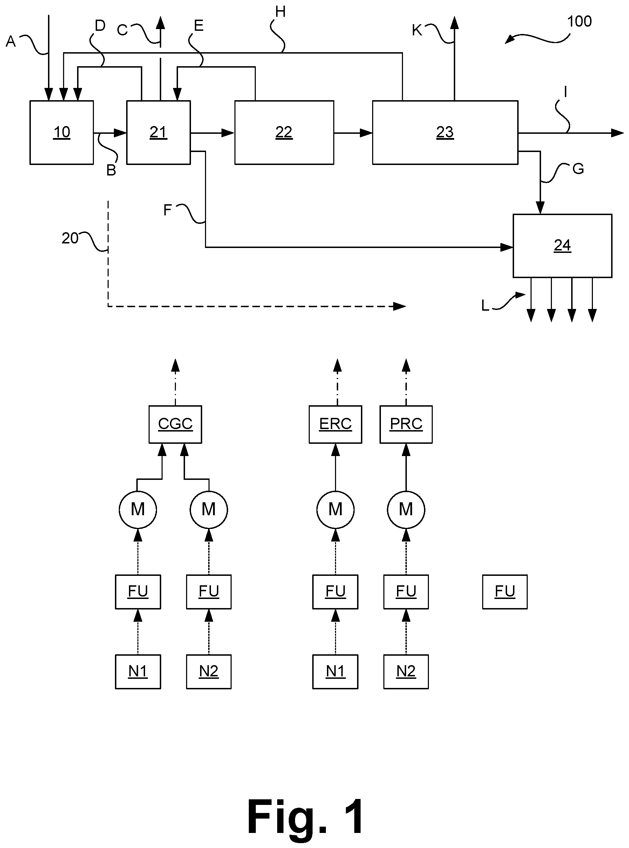

In Figur 1 ist ein Verfahren gemäß einer Ausgestaltung der vorliegenden Erfindung in Form eines vereinfachten Prozessflussdiagramms veranschaulicht und insgesamt mit 100 bezeichnet. Wenngleich die nachfolgenden Erläuterungen sich auf ein Verfahren und entsprechende Verfahrensschritte beziehen, gelten diese für eine entsprechende Anlage und deren Komponenten in gleicher Weise.

In dem Verfahren 100 werden ein oder mehrere Cracker (Spaltöfen) 10 eingesetzt, die mit einem Feed A (sowie Dampf, wie hier nicht gesondert veranschaulicht) beschickt werden, und dem oder denen ein Rohgas B entnommen wird.

Das Rohgas B wird zumindest zum Teil einer hier insgesamt mit 20 bezeichneten Aufbereitung unterworfen, die in an sich bekannter Weise einen Quenchschritt 21 unter Abscheidung von Pyrolyseöl C sowie eine Verdichtung (Rohgasverdichtung) 22 und eine thermische Trennung 23 unter Verwendung eines C2-Kältemittels und eines C3-Kältemittels umfasst. In dem Quenchschritt 21 kann Dampf D bereitgestellt und in den oder die Cracker 10 zurückgeführt werden. In der Verdichtung 22 kann Pyrolysebenzin E abgetrennt und beispielsweise in den Quenchschritt 21 zurückgeführt werden. In dem Quenchschritt 21 können ferner Kohlenwasserstoffe F mit drei und mehr Kohlenstoffatomen abgetrennt und in einen entsprechenden Verarbeitungsschritt 24 überführt werden, in dem auch entsprechende Kohlenwasserstoffe G aus der thermischen Trennung 23 eingespeist werden können. In der thermischen Trennung 23 kann ein Ethanstrom H bereitgestellt und in den oder die Spaltöfen 10 zurückgeführt werden. Ein Ethylenstrom I kann als Produkt ausgeführt werden. Sogenanntes Tailgas K, das insbesondere Methan und Wasserstoff enthält, wird ausgeschleust. Weitere Produktströme, insgesamt mit L bezeichnet, werden in dem weiteren Verarbeitungsschritt 24 bereitgestellt.

Wie hier symbolisch in einem unteren Bereich der Figur 1 veranschaulicht, wird für die Rohgasverdichtung 22 ein mit CGC bezeichneter Verdichter verwendet, das Ethylenkältemittel wird unter Verwendung eines mit ERC bezeichneten C2-Kältemittelverdichters verdichtet und das Propylenkältemittel wird unter Verwendung eines mit PRC bezeichnetenC3-Kältemittelverdichters verdichtet. Der Rohgasverdichter CGC, der C2-Kältemittelverdichter ERC und der C3-Kältemittelverdichter PRC werden jeweils zumindest zu einem Teil unter Verwendung eines oder mehrerer elektrischer Antriebe M betrieben.

Genauer umfasst in der hier veranschaulichten Ausgestaltung der Erfindung der Rohgasverdichter CGC zwei serielle Verdichterstränge (nicht gesondert dargestellt), wobei jeder der Verdichterstränge des Rohgasverdichters CGC sowie der C2-Kältemittelverdichter ERC und der C3-Kältemittelverdichter PRC jeweils mittels zumindest teilweise identische Leistungsmerkmale aufweisender elektrischer Antriebe betrieben werden. Diese können hier als baugleiche drehzahlvariable Antriebe bereitgestellt werden und jeweils über einen Frequenzumrichter FU gespeist werden. Insgesamt sind dabei in dem hier veranschaulichten Beispiel fünf Frequenzumrichter FU bereitgestellt, von denen zu jedem Zeitpunkt zwei (im paarweisen Volllast-Wechselbetrieb) oder vier (im paarweisen Teillast-Betrieb) zur Speisung der elektrischen Antriebe M eingesetzt werden und einer aus Redundanzgründen bereitgehalten wird. Im hier veranschaulichten Beispiel werden die Frequenzumrichter FU in der dargestellten Weise an unterschiedliche Netze bzw. Netzteile N1, N2 angebunden. Die Erfindung ist durch das hier veranschaulichte Beispiel nicht beschränkt.

Der Dampf D oder anderer in dem Verfahren bereitgestellter Dampf kann insbesondere Höchstdruckdampf umfassen, der exportiert und/oder zumindest teilweise ohne Verwendung zum Antrieb des Rohgasverdichters RGC, des C2-Kältemittelverdichters ERC und des C3-Kältemittelverdichters PRC als Wärmequelle für andere Verfahrensschritte verwendet wird. Der Höchstdruckdampf kann einer hier allgemein mit 50 angegebenen Adaptionseinheit hinsichtlich Druck und/oder Temperatur für den Export und/oder den Verwendungszweck angepasst werden. In der der Adaptionseinheit 50 anfallende Kondensationswärme kann in der erläuterten Weise genutzt werden.

Zu weiteren Ausgestaltungen der Erfindung, die alle auch in dem in Figur 1 dargestellten Verfahren 100 einsetzbar sind, sei auf die obigen Erläuterungen nochmals ausdrücklich verwiesen.

## Patentansprüche

1. Verfahren (100) zur Herstellung von Ethylen und/oder anderen Olefinen durch Steamcracken, bei dem ein oder mehrere Cracker (10) mit einem paraffinhaltigen Feed beschickt werden und dem einen oder mehreren Crackern (10) ein Rohgas entnommen wird, wobei das Rohgas zumindest zum Teil einer Aufbereitung (20) unterworfen wird, die eine Rohgasverdichtung (22) sowie eine thermische Trennung (23) unter Verwendung eines C2-Kältemittels und eines C3-Kältemittels umfasst, wobei für die Rohgasverdichtung (22) ein Rohgasverdichter (CGC) verwendet wird, wobei das Ethylenkältemittel unter Verwendung eines C2-KältemittelverdichterS (ERC) verdichtet wird, und wobei das Propylenkältemittel unter Verwendung eines C3-KältemittelverdichterS (PRC) verdichtet wird, **dadurch gekennzeichnet, dass** der Rohgasverdichter (CGC), der C2-Kältemittelverdichter (ERC) und der C3-Kältemittelverdichter (PRC) jeweils zumindest zu einem Teil unter Verwendung eines oder mehrerer elektrischer Antriebe (M) betrieben werden.

2. Verfahren nach Anspruch 1, bei dem unter Verwendung von Abwärme Höchstdruckdampf bereitgestellt und exportiert und/oder zumindest teilweise ohne Verwendung zum Antrieb des Rohgasverdichters (RGC), des C2-Kältemittelverdichters (ERC) und des C3-Kältemittelverdichters (PRC) als Wärmequelle für andere Verfahrensschritte verwendet wird.

3. Verfahren nach Anspruch 2, bei dem der Höchstdruckdampf in einer Adaptionseinheit (50) hinsichtlich Druck und/oder Temperatur für den Export und/oder den Verwendungszweck angepasst wird.

4. Verfahren nach Anspruch 3, wobei in der Adaptionseinheit (50) anfallende Kondensationswärme genutzt wird.

5. Verfahren nach einem der vorstehenden Ansprüche, das ohne Verwendung eines Hochdruckdampfkessels betrieben wird.

6. Verfahren nach einem der vorstehenden Ansprüche, bei dem der Rohgasverdichter (CGC) zwei serielle Verdichterstränge umfasst, wobei jeder der Verdichterstränge des Rohgasverdichters (CGC) sowie der C2-Kältemittelverdichter (ERC) und der C3-Kältemittelverdichter (PRC) jeweils mittels zumindest teilweise identische Leistungsmerkmale aufweisender elektrischer Antriebe betrieben werden.

7. Verfahren nach Anspruch 6, bei dem die zumindest teilweise identische Leistungsmerkmale aufweisenden elektrischen Antriebe als baugleiche drehzahlvariable Antriebe bereitgestellt werden und jeweils über Frequenzumrichter (FU) gespeist werden.

8. Verfahren nach Anspruch 7, bei dem vier Frequenzumrichter (FU) bereitgestellt sind, von denen zu jedem Zeitpunkt zwei oder vier zur Speisung der elektrischen Antriebe eingesetzt werden, und bei dem insbesondere ein fünfter Frequenzumrichter (FU) aus Redundanzgründen bereitgehalten wird.

9. Verfahren nach einem der vorstehenden Ansprüche, bei dem der Rohgasverdichter (CGC), der C2-Kältemittelverdichter (ERC) und der C3-Kältemittelverdichter (PRC) ebenerdig aufgestellt sind.

10. Verfahren nach einem der vorstehenden Ansprüche, bei dem in dem Rohgasverdichter (CGC) eine Zwischenkühlung zumindest zeitweise unter Verwendung von Fremdkälte vorgenommen wird.

11. Verfahren nach Anspruch 10, bei dem die Fremdkälte für die Zwischenkühlung unter Verwendung zumindest eines Teils des C2-Kältemittels und/oder des C3-Kältemittels bereitgestellt wird.

12. Verfahren nach einem der Ansprüche 10 oder 11, bei dem die Zwischenkühlung dann unter Verwendung von Fremdkälte vorgenommen wird, wenn eine Kühlwassertemperatur und/oder Wassereinspritzung als nicht ausreichend und/oder nicht funktionsfähig erkannt wird.

13. Verfahren nach einem der vorstehenden Ansprüche, bei dem Antriebsleistungen der elektrischen Antriebe des Rohgasverdichters (CGC), des C2-Kältemittelverdichter (ERC) und des C3-Kältemittelverdichters (PRC) durch eine Verschiebung von Kälteleistung zwischen einem Ethylenkältemittelkreislauf, in dem das Ethylenkältemittel eingesetzt wird, und eines Propylenkältemittelkreislaufs, in dem das Propylenkältemittel eingesetzt wird, und/oder Einbindung von Propylenkälte als zusätzliche Zwischenkühlung des Rohgasverdichters (CGC) eingestellt werden.

14. Anlage zur Herstellung von Ethylen und/oder anderen Olefinen durch Steamcracken, mit einem oder mehreren Cracker (10), die zur Beschickung mit einem paraffinhaltigen Feed und zur Bereitstellung eines Rohgases eingerichtet sind, wobei die Anlage dafür eingerichtet ist, das Rohgas zumindest zum Teil einer Aufbereitung (20) zu unterwerfen, die eine Verdichtung (22) und eine thermische Trennung (23) unter Verwendung eines C2-Kältemittels und eines C3-Kältemittels umfasst, wobei für die Rohgasverdichtung (22) ein Rohgasverdichter (CGC) bereitgestellt ist, zur Verdichtung des C2-Kältemittels ein C2-Kältemittelverdichter (ERC) bereitgestellt ist, und zur Verdichtung des C3-Kältemittels ein C3-Kältemittelverdichter (PRC) bereitgestellt ist, **dadurch gekennzeichnet, dass** zur Bereitstellung jeweils zumindest eines Teils der Antriebsleistung des Rohgasverdichters (CGC), des C2-Kältemittelverdichters (ERC) und des C3-Kältemittelverdichter (PRC) jeweils ein elektrischer Hauptantrieb (M) bereitgestellt ist.

15. Anlage nach Anspruch 14, die zur Durchführung eines Verfahrens nach einem der vorstehenden Ansprüche eingerichtet ist.
